(19) Europäisches Patentamt
European Patent Office
Office européen des brevets

(11) **EP 4 715 838 A1**

(12) **EUROPEAN PATENT APPLICATION**

(43) Date of publication:
**25.03.2026 Bulletin 2026/13**

(21) Application number: **25202919.4**

(22) Date of filing: **18.09.2025**

(51) International Patent Classification (IPC):
***G16H 30/40*** *(2018.01)*

(52) Cooperative Patent Classification (CPC):
**G16H 30/40; G16H 50/30;** G16H 50/20

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **23.09.2024 US 202418892888**

(71) Applicant: **Siemens Healthineers AG**
**91301 Forchheim (DE)**

(72) Inventors:
• **ZHANG, Yanbo**
**Plainsboro, NJ 08536 (US)**
• **ADIYOSO, Arnaud Arindra**
**90431 Nürnberg (DE)**

• **GEORGESCU, Bogdan**
**Princeton, NJ 08540 (US)**
• **BALACHANDRAN, Abishek**
**600010 Chennai, Tamil Nadu (IN)**
• **GEIGER, Bernhard**
**Cranbury, NJ 08512 (US)**
• **SPERL, Jonathan**
**96047 Bamberg (DE)**
• **GHESU, Florin-Cristian**
**91083 Baiersdorf (DE)**
• **GRBIC, Sasa**
**Plainsboro, NJ, 08536 (US)**
• **COMANICIU, Dorin**
**Princeton, NJ 08540 (US)**

(74) Representative: **HKW Intellectual Property PartG mbB**
**Theresienhöhe 12**
**80339 München (DE)**

(54) **ADVANCED PULMONARY NODULE ANALYSIS SYSTEM WITH MALIGNANCY-WEIGHTED DETECTION AND CUSTOMIZED VISUALIZATION**

(57) Systems and methods for advanced pulmonary nodule analysis with malignancy - weighted detection and customized visualization are provided. One or more medical images of a patient are received. One or more candidate nodules are detected in the one or more medical images. Each of the one or more candidate nodules are associated with a nodule detection score. A malignancy score is determined for each of the one or more candidate nodules. The nodule detection score is weighted for each of the one or more candidate nodules based on its malignancy score. The weighted nodule detection scores are output.

FIG. 1

100

Receive one or more medical images of a patient
102

Detect one or more candidate nodules in the one or more medical images, each of the one or more candidate nodules associated with a nodule detection score
104

Determine a malignancy score for each of the one or more candidate nodules
106

Weight the nodule detection score for each of the one or more candidate nodules based on its malignancy score
108

Output the weighted nodule detection scores
110

**Description**

TECHNICAL FIELD

The present invention relates generally to medical imaging analysis, and in particular to an advanced pulmonary nodule analysis system with malignancy-weighted detection and customized visualization.

BACKGROUND

Pulmonary nodule analysis involves the detection of nodules in medical images of the chest of a patient and classifying the detected nodules as being malignant or benign. Early detection of pulmonary nodules is associated with improved prognosis, greater treatment options, and reduced risk of metastasis. Conventionally, computer-aided and AI-based approaches have been proposed for detecting pulmonary nodules in medical images. However, such conventional pulmonary nodule detection approaches have a number of limitations. For instance, conventional pulmonary nodule detection approaches lack integration with type classification, malignancy assessment, and personalized visualization systems for providing a unified platform. This fragmentation can compromise efficiency, consistency, and accuracy in pulmonary nodule analysis. Additionally, conventional pulmonary nodule detection approaches have lower accuracy of high-risk nodules, which may result in clinicians spending resources and time in analyzing low-risk nodules while delaying analysis and treatment of high-risk nodules. Further, conventional pulmonary nodule detection approaches provide static visualization interfaces and are not tailored to a clinician's preferences of clinical needs.

BRIEF SUMMARY OF THE INVENTION

In accordance with one or more embodiments, apparatuses, systems, methods, and computer-program products for advanced pulmonary nodule analysis with malignancy-weighted detection and customized visualization are provided. One or more medical images of a patient are received. One or more candidate nodules are detected in the one or more medical images. Each of the one or more candidate nodules are associated with a nodule detection score. A malignancy score is determined for each of the one or more candidate nodules. The nodule detection score is weighted for each of the one or more candidate nodules based on its malignancy score. The weighted nodule detection scores are output.

In one embodiment, for each respective candidate nodule of the one or more candidate nodules, a nodule type score is determined for each of a plurality of nodule types for the respective candidate nodule. The malignancy is determined for the respective candidate nodule based on the nodule type scores for the respective candidate nodule. In one embodiment, a size of each of the one or more candidate nodules is determined and the malignancy is determined for the respective candidate nodule further based on the size of the respective candidate nodule. In one embodiment, the plurality of nodule types comprises one or more of solid nodule, part-solid nodule, non-solid nodule, calcified nodule, perifissural nodule, or non-nodule.

In one embodiment, user input is received defining visualization settings for presenting the one or more candidate nodules. The one or more candidate nodules are filtered according to the visualization settings. The one or more filtered candidate nodules are displayed. The visualization settings comprise at least one of: one or more thresholds of the weighted nodule detection scores, one or more thresholds of a nodule size, a selection of one or more nodule types, or one or more thresholds of a malignancy scores.

In one embodiment, the one or more candidate nodules comprises one or more candidate pulmonary nodules.

These and other advantages of the invention will be apparent to those of ordinary skill in the art by reference to the following detailed description and the accompanying drawings.

BRIEF DESCRIPTION OF THE DRAWINGS

Figure 1 shows a method for detecting nodules in medical images, in accordance with one or more embodiments;

Figure 2 shows a workflow for detecting nodules in medical images, in accordance with one or more embodiments;

Figure 3 shows a user interface for defining visualization settings for viewing candidate nodules, in accordance with one or more embodiments;

Figure 4 shows an exemplary artificial neural network that may be used to implement one or more embodiments;

Figure 5 shows a convolutional neural network that may be used to implement one or more embodiments;

Figure 6 shows a schematic structure of a recurrent machine learning model that may be used to implement one or more embodiments; and

Figure 7 shows a high-level block diagram of a computer that may be used to implement one or more embodiments.

DETAILED DESCRIPTION

The present invention generally relates to methods and systems for advanced pulmonary nodule analysis with malignancy-weighted detection and customized visualization. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for pulmonary nodule analysis with malignancy-weighted detection. Advantageously, by weighting detection of pulmonary nodules based on malignancy, detected pulmonary nodules that have a higher probability of being malignant are highlighted for further analysis, e.g., by downstream nodule analysis systems or by clinicians. Embodiments described herein further provide for a customized visualization of data on the detected pulmonary nodules to meet diverse clinical needs. Embodiments described herein thus address the limitations of conventional pulmonary nodule detection approaches by providing an integrated, malignancy-weighted, and customizable nodule analysis framework, streamlining the clinical decision-making process.

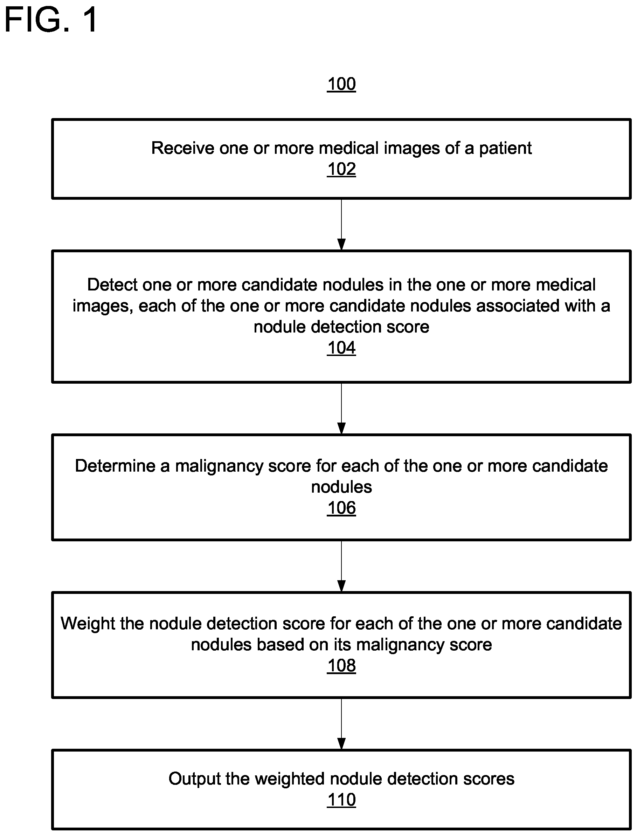

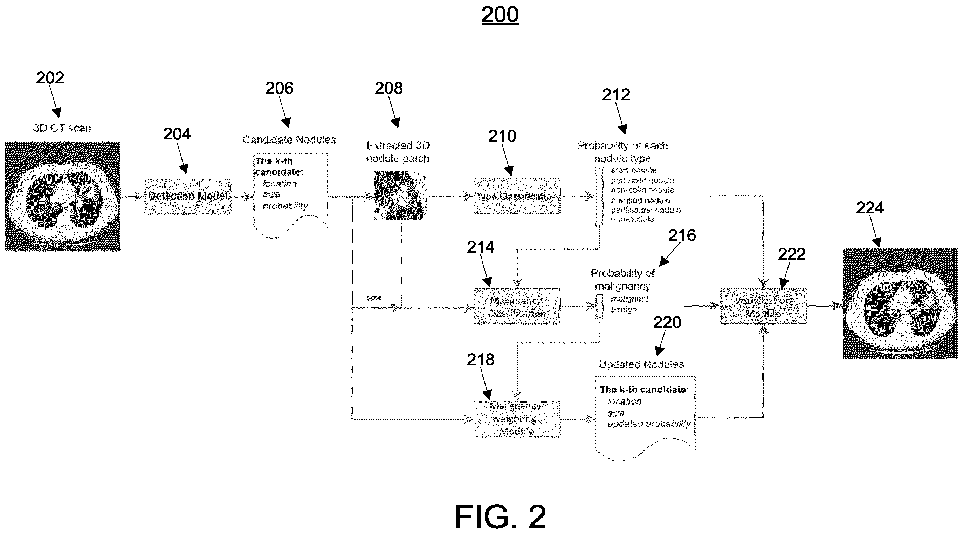

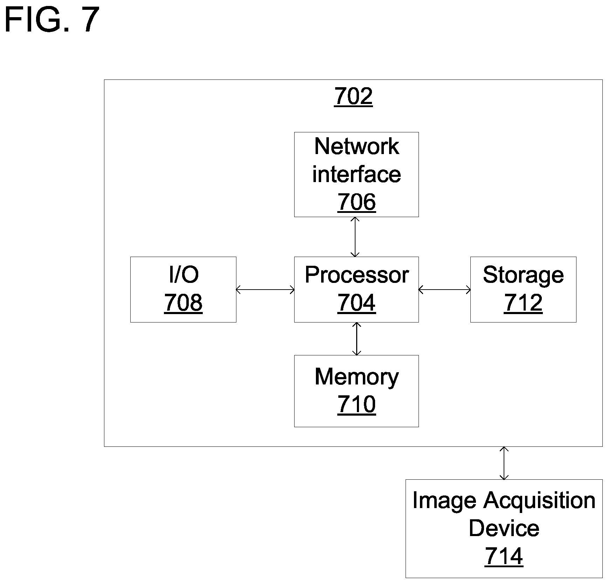

Figure 1 shows a method 100 for detecting nodules in medical images, in accordance with one or more embodiments. The steps and sub-steps of method 100 may be performed by one or more suitable computing devices, such as, e.g., computer 702 of Figure 7. Figure 2 shows a workflow 200 for detecting nodules in medical images, in accordance with one or more embodiments. Figure 1 and Figure 2 will be described together.

At step 102 of Figure 1, one or more medical images of a patient are received. In one embodiment, the one or more medical images depict a chest of the patient. However, the one or more medical images may depict any other suitable anatomical object of interest of the patient, such as, e.g., other organs, vessels, bones, etc.

In one embodiment, the one or more medical images are CT (computed tomography) images. For example, as shown in workflow 200 of Figure 2, the one or more medical images may comprise 3D (three dimensional) CT scan 202. However, the one or more medical images may comprise any other suitable modality, such as, e.g., MRI (magnetic resonance imaging), US (ultrasound), x-ray, or any other medical imaging modality or combinations of medical imaging modalities. The one or more medical images may be 2D (two dimensional) images and/or 3D volumes, and may comprise a single medical image or a plurality of medical images.

The one or more medical images may be received, for example, by directly receiving the one or more medical images from the image acquisition device (e.g., image acquisition device 714 of Figure 7) as the one or more medical images are acquired, by loading the one or more medical images from a storage or memory of a computer system (e.g., storage 712 or memory 710 of computer 702 of Figure 7), or by receiving the one or more medical images from a remote computer system (e.g., computer 702 of Figure 7). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, one or more candidate nodules are detected in the one or more medical images. Each of the one or more candidate nodules are associated with a nodule detection score. The one or more candidate nodules are detected objects in the one or more medical images that are candidates to be nodules. As used herein, the nodules may also comprise tumors, lesions, and/or any other abnormality.

The one or more candidate nodules are detected using a machine learning based detection model. For example, as shown in workflow 200 of Figure 2, candidate nodules 206 are detected in 3D CT scan 202 by detection model 204. The one or more candidate nodules may be detected from the one or more medical images using the machine learning detection model according to any suitable (e.g., well-known) approach. In some embodiments, the one or more candidate nodule may be detected according to U.S. Patent Application No. 18/629,023, entitled "Computer-Aided Diagnosis System for Pulmonary Nodule Analysis using PCCT Images," the disclosure of which is incorporated herein by reference in its entirety. The machine learning based detection model receives as input the one or more medical images and generates as output the one or more candidate nodules each defined by a location, a size, and the nodule detection score.

The nodule detection score represents a probability or confidence that the detected object in the one or more medical images is a nodule. The nodule detection score may be represented in any suitable format. For example, the nodule detection score may be represented as a value between 0 and 1, where a nodule detection score of 0 represents a 0% probability that the object is a nodule and a nodule detection score of 1 represents a 100% probability that the object is a nodule.

At step 106 of Figure 1, a malignancy score is determined for each of the one or more candidate nodules. The malignancy score represents a probability that a candidate nodule is malignant (or inversely benign).

To determine the malignancy score of each respective one of the one or more candidate nodules, a patch of the respective candidate nodule is first extracted from the one or more medical images. The extracted patch may be, for example, centered around the respective candidate nodule and have a predefined size. In one example, as shown in workflow 200 of Figure 2, extracted 3D nodule patches 208 of candidate nodules 206 are extracted from 3D CT scan 202.

A type of the respective candidate nodule is then determined from the extracted patch using a machine learning based type classification model. For example, as shown in workflow 200 of Figure 2, a type 212 of a respective candidate nodule 206 is determined from the extracted 3D nodule patch 208 by type classification model 210. The type of the respective candidate nodule may be determined from the extracted patch using the machine learning based type classification model according to any suitable (e.g., well-known) approach. In some embodiments, the type of the respective candidate nodule may be determined according to U.S. Patent Application Publication No. 2023/0351601, entitled "Classifying a Lesion Based on Longitudinal Studies," or U.S. Patent Application Publication No. 2024/0046466, entitled "Determining Characteristics of Adipose Tissue using Artificial Neural Network," or U.S. Patent Application No. 18/629,023, entitled "Computer-Aided Diagnosis System for Pulmonary Nodule Analysis using PCCT Images," the disclosures of which are incorporated herein by reference in their entirety.

The machine learning based type classification model receives as input the extracted patch and generates as output a classification of a type of the respective candidate nodule. The type of the respective candidate nodule may comprise, for example, solid nodule, part-solid nodule, non-solid nodule, calcified nodule, perifissural nodule, and non-nodule. The classification of the type of the respective candidate nodule may comprise a nodule type score for each of a plurality of types of nodules. The nodule type score represents a probability that the respective candidate nodule is of a particular type. The nodule type score may be represented in any suitable format (e.g., a value between 0 and 1).

The malignancy score of the respective candidate nodule is then determined from the extracted patch using a machine learning based malignancy classification model based on the classification of the type of the respective candidate nodule and the size of the respective candidate nodule (determined by the machine learning based detection model at step 104 of Figure 1). For example, as shown in workflow 200 of Figure 2, a malignancy 216 of the respective candidate nodule 206 is determined from the extracted 3D nodule patch 208 by malignancy classification model 214 based on type 212 of the respective candidate nodule 206 and a size of the respective candidate nodule 206 (determined by detection model 204). The malignancy of the respective candidate nodule may be determined from the extracted patch using the machine learning based malignancy classification model according to any suitable (e.g., well-known) approach. In some embodiments, the malignancy score of the respective candidate nodule may be determined according to U.S. Patent Application No. 18/629,023, entitled "Computer-Aided Diagnosis System for Pulmonary Nodule Analysis using PCCT Images," the disclosure of which is incorporated herein by reference in its entirety. The machine learning based malignancy classification model receives as input the extracted patch, the classification of the type of the respective candidate nodules (i.e., the nodule type scores), and the size of the respective candidate nodule and generates as output a classification of a malignancy of the respective candidate nodule defining the malignancy score of the respective candidate nodule. The malignancy score may be represented in any suitable format (e.g., a value between 0 and 1).

At step 108 of Figure 1, the nodule detection scores are weighted based on their malignancy scores. In one example, as shown in workflow 200 of Figure 2, the probability associated with candidate nodules 206 are weighted by malignancy-weighting module 218 based on malignancy 216 to provide an updated candidate nodules 220 defining an updated probability (i.e., weighted nodule detection scores) as well as the locations and sizes (as determined by detection model 204).

In one embodiment, the nodule detection scores may be weighted according to the weighting equation of Equation (1):

$$p' = \frac{(m + c)^{\gamma}}{(1 + c)^{\gamma}} p \qquad (1)$$

where *p'* represents the weighted nodule detection score, *p* represents the nodule detection score, *m* represents the malignancy score, and *c* and $\gamma$ are parameters for regulating the weight adjustment.

The nodule detection scores may be weighted according to any other suitable approach. For example, in one embodiment, the nodule detection scores may be weighted using machine learning based methods, classification methods, or weighting methods.

At step 110 of Figure 1, the weighted nodule detection scores are output. In some embodiments, the locations and/or sizes of the one or more candidate nodules are also output. For example, the locations, sizes, and/or weighted nodule detection scores for the one or more candidate nodules can be output by displaying the locations, sizes, and/or weighted nodule detection scores on a display device of a computer system (e.g., I/O 708 of computer 702 of Figure 7), storing the locations, sizes, and/or weighted nodule detection scores on a memory or storage of a computer system (e.g., memory 710 or storage 712 of computer 702 of Figure 7), or by transmitting the locations, sizes, and/or weighted nodule detection scores to a remote computer system (e.g., computer 702 of Figure 7).

In one embodiment, data on the one or more candidate nodules are consolidated and presented according to user defined criteria. In one example, as shown in workflow 200 of Figure 2, visualization module 222 may generate customized visualization 224 presenting types 212 of the candidate nodules 206, malignancy 216 of the candidate nodules 206, and the updated candidate nodules 220 defined by location, size, and weighted nodule detection scores.

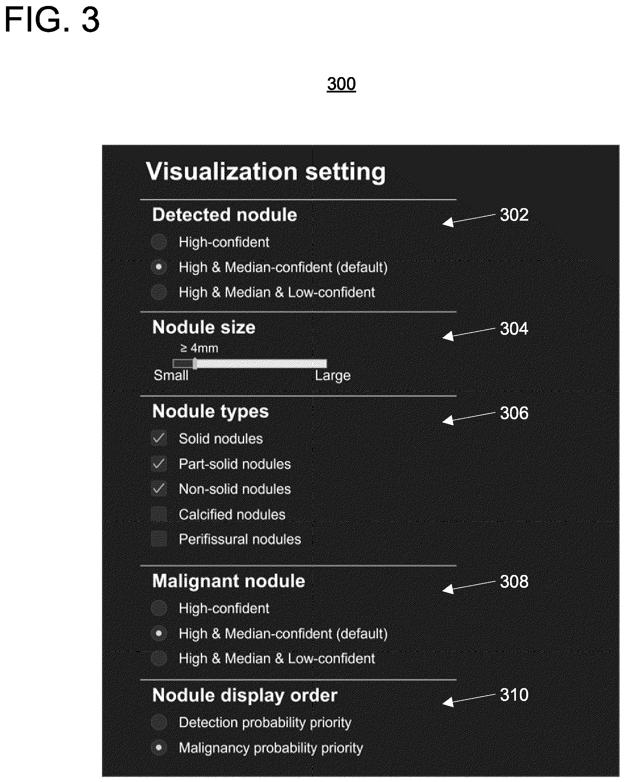

Figure 3 shows a user interface 300 for defining visualization settings for viewing the one or more candidate nodules, in accordance with one or more embodiments. A user may interact with user interface 300 via, e.g., a touchscreen, a mouse, a keyboard, or any other suitable device (e.g., I/O 708 of Figure 7) for defining the criteria to filter the one or more candidate nodules. The one or more candidate nodules are filtered according to the visualization settings. Detected nodule settings 302 enable a user to filter the one or more candidate nodules to view high-confident detected candidate nodules, high & median-confident detected candidate nodules, or high & median & low-confident detected candidate nodules. The high, median, and low-confidence settings are each associated with a threshold of the weighted nodule detection scores of the one or more candidate modules. Nodule size settings 304 enable the user to define one or more thresholds to filter the one or more candidate nodules to view candidate nodules according to nodule size. Nodule types settings 306 enable a user to filter the one or more candidate nodules according to one or more nodule types. Malignant nodule settings 308 enable a user to filter the one or more candidate nodules to view high-confident malignant candidate nodules, high & median-confident malignant candidate nodules, or high & median & low-confident malignant candidate nodules. The high, median, and low-confidence settings are each associated with a threshold of the malignancy scores of the one or more candidate modules. Nodule display order settings 310 enable the user to order the filtered candidate nodules according to detection probability priority (e.g., in ascending or descending order of the weighted nodule detection scores) or malignancy probability priority (e.g., in ascending or descending order of the malignancy scores). Advantageously, user interface 300 enables a user to quickly and easily identify and analyze candidate nodules of the highest concern or interest.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the machine learning models utilized at steps 104, 106, and 108 of Figure 1 and detection model 204, type classification model 210, and malignancy classification model 214 of Figure 2, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

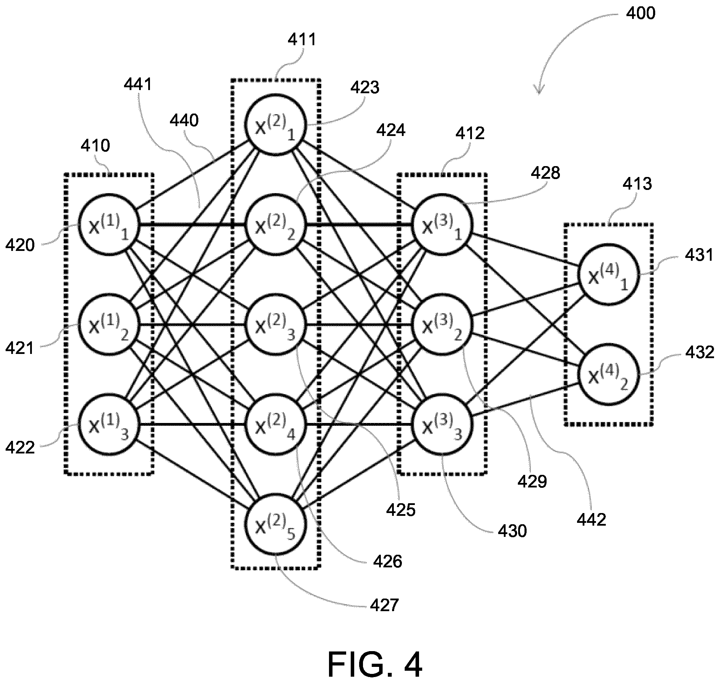

Figure 4 shows an embodiment of an artificial neural network 400 that may be used to implement one or more machine learning models described herein. Alternative terms for "artificial neural network" are "neural network", "artificial neural net" or "neural net".

The artificial neural network 400 comprises nodes 420, ..., 432 and edges 440, ..., 442, wherein each edge 440, ..., 442 is a directed connection from a first node 420, ..., 432 to a second node 420, ..., 432. In general, the first node 420, ..., 432 and the second node 420, ..., 432 are different nodes 420, ..., 432, it is also possible that the first node 420, ..., 432 and the second node 420, ..., 432 are identical. For example, in Figure 4 the edge 440 is a directed connection from the node 420 to the node 423, and the edge 442 is a directed connection from the node 430 to the node 432. An edge 440, ..., 442 from a first node 420, ..., 432 to a second node 420, ..., 432 is also denoted as "ingoing edge" for the second node 420, ..., 432 and as "outgoing edge" for the first node 420, ..., 432.

In this embodiment, the nodes 420, ..., 432 of the artificial neural network 400 can be arranged in layers 410, ..., 413, wherein the layers can comprise an intrinsic order introduced by the edges 440, ..., 442 between the nodes 420, ..., 432. In particular, edges 440, ..., 442 can exist only between neighboring layers of nodes. In the displayed embodiment, there is an input layer 410 comprising only nodes 420, ..., 422 without an incoming edge, an output layer 413 comprising only nodes 431, 432 without outgoing edges, and hidden layers 411, 412 in-between the input layer 410 and the output layer 413. In general, the number of hidden layers 411, 412 can be chosen arbitrarily. The number of nodes 420, ..., 422 within the input layer 410 usually relates to the number of input values of the neural network, and the number of nodes 431, 432 within the output layer 413 usually relates to the number of output values of the neural network.

In particular, a (real) number can be assigned as a value to every node 420, ..., 432 of the neural network 400. Here, $x^{(n)}_i$ denotes the value of the i-th node 420, ..., 432 of the n-th layer 410, ..., 413. The values of the nodes 420, ..., 422 of the input layer 410 are equivalent to the input values of the neural network 400, the values of the nodes 431, 432 of the output layer 413 are equivalent to the output value of the neural network 400. Furthermore, each edge 440, ..., 442 can comprise a weight being a real number, in particular, the weight is a real number within the interval [-1, 1] or within the interval [0, 1]. Here, $w^{(m,n)}_{i,j}$ denotes the weight of the edge between the i-th node 420, ..., 432 of the m-th layer 410, ..., 413 and the j-th node 420, ..., 432 of the n-th layer 410, ..., 413. Furthermore, the abbreviation $w^{(n)}_{i,j}$ is defined for the weight $w^{(n,n+1)}_{i,j}$.

In particular, to calculate the output values of the neural network 400, the input values are propagated through the neural network. In particular, the values of the nodes 420, ..., 432 of the (n+1)-th layer 410, ..., 413 can be calculated based on the values of the nodes 420, ..., 432 of the n-th layer 410, ..., 413 by

$$\mathrm{x}^{(n+1)_j} = \mathrm{f}\left(\sum_i \mathrm{x}^{(n)_i} \cdot w^{(n)_{i,j}}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 410 are given by the input of the neural network 400, wherein values of the first hid-den layer 411 can be calculated based on the values of the input layer 410 of the neural network, wherein values of the second hidden layer 412 can be calculated based in the values of the first hidden layer 411, etc.

In order to set the values $w^{(m,n)i,j}$ for the edges, the neural network 400 has to be trained using training data. In particular, training data comprises training input data and training output data (denoted as $t_i$). For a training step, the neural network 400 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 400 (backpropagation algorithm). In particular, the weights are changed according to

$$\mathrm{w}'^{(n)_{i,j}} = \mathrm{w}^{(n)_{i,j}} - \gamma \cdot \delta^{(n)_j} \cdot \mathrm{x}^{(n)_i}$$

wherein y is a learning rate, and the numbers $\delta^{(n)}_j$ can be recursively calculated as

$$\delta^{(n)j} = \left(\sum_k \delta^{(n+1)k} \cdot w^{(n+1)j,k}\right) \cdot f'\left(\sum_i x^{(n)i} \cdot w^{(n)i,j}\right)$$

based on $\delta^{(n+1)}{}_j$, if the (n+1)-th layer is not the output layer, and

$$\delta^{(n)j} = \left(x^{(n+1)j} - t^{(n+1)j}\right) \cdot f'\left(x^{(n)i} \cdot w^{(n)i,j}\right)$$

if the (n+1)-th layer is the output layer 413, wherein f is the first derivative of the activation function, and $t^{(n+1)}{}_j$ is the comparison training value for the j-th node of the output layer 413.

A convolutional neural network is a neural network that uses a convolution operation instead of general matrix multiplication in at least one of its layers (so-called "convolutional layer"). In particular, a convolutional layer performs a dot product of one or more convolution kernels with the convolutional layer's input data / image, wherein the entries of the one or more convolution kernels are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

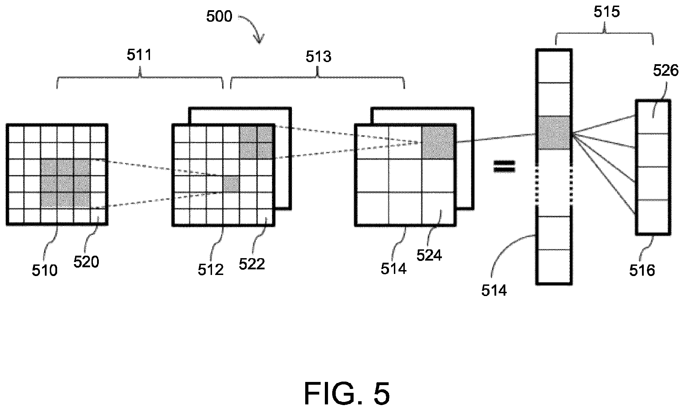

Figure 5 shows an embodiment of a convolutional neural network 500 that may be used to implement one or more machine learning models described herein. In the displayed embodiment, the convolutional neural network 500 comprises an input node layer 510, a convolutional layer 511, a pooling layer 513, a fully connected layer 514 and an output node layer 516, as well as hidden node layers 512, 514. Alternatively, the convolutional neural network 500 can comprise several convolutional layers 511, several pooling layers 513 and several fully connected layers 515, as well as other types of layers. The order of the layers can be chosen arbitrarily, usually fully connected layers 515 are used as the last layers before the output layer 516.

In particular, within a convolutional neural network 500 nodes 520, 522, 524 of a node layer 510, 512, 514 can be considered to be arranged as a d-dimensional matrix or as a d-dimensional image. In particular, in the two-dimensional case the value of the node 520, 522, 524 indexed with i and j in the n-th node layer 510, 512, 514 can be denoted as x(n)[i, j]. However, the arrangement of the nodes 520, 522, 524 of one node layer 510, 512, 514 does not have an effect on the calculations executed within the convolutional neural network 500 as such, since these are given solely by the structure and the weights of the edges.

A convolutional layer 511 is a connection layer between an anterior node layer 510 (with node values x(n-1)) and a posterior node layer 512 (with node values x(n)). In particular, a convolutional layer 511 is characterized by the structure and the weights of the incoming edges forming a convolution operation based on a certain number of kernels. In particular, the structure and the weights of the edges of the convolutional layer 511 are chosen such that the values x(n) of the nodes 522 of the posterior node layer 512 are calculated as a convolution x(n) = K * x(n-1) based on the values x(n-1) of the nodes 520 anterior node layer 510, where the convolution * is defined in the two-dimensional case as

$$x_k^{(n)}[i,j] = \left(K * x^{(n-1)}\right)[i,j] = \sum_{i'}\sum_{j'} K[i',j'] \cdot x^{(n-1)}[i-i', j-j'].$$

Here the kernel K is a d-dimensional matrix (in this embodiment, a two-dimensional matrix), which is usually small compared to the number of nodes 520, 522 (e.g., a 3x3 matrix, or a 5x5 matrix). In particular, this implies that the weights of the edges in the convolution layer 511 are not independent, but chosen such that they produce said convolution equation. In particular, for a kernel being a 3x3 matrix, there are only 9 independent weights (each entry of the kernel matrix corresponding to one independent weight), irrespectively of the number of nodes 520, 522 in the anterior node layer 510 and the posterior node layer 512.

In general, convolutional neural networks 500 use node layers 510, 512, 514 with a plurality of channels, in particular, due to the use of a plurality of kernels in convolutional layers 511. In those cases, the node layers can be considered as (d+1)-dimensional matrices (the first dimension indexing the channels). The action of a convolutional layer 511 is then a two-dimensional example defined as

$$x^{(n)}{}_b[i,j] = \sum_a K_{a,b} * x^{(n-1)a}[i,j] = \sum_a \sum_{i'} \sum_{j'} K_{a,b}[i',j'] \cdot x^{(n-1)a}[i-i', j-j']$$

where $x^{(n-1)a}$ corresponds to the a-th channel of the anterior node layer 510, $x^{(n)b}$ corresponds to the b-th channel of the posterior node layer 512 and $K_{a,b}$ corresponds to one of the kernels. If a convolutional layer 511 acts on an anterior node layer 510 with A channels and outputs a posterior node layer 512 with B channels, there are A·B independent d-dimensional kernels $K_{a,b}$.

In general, in convolutional neural networks 500 activation functions are used. In this embodiment ReLU (acronym for "Rectified Linear Units") is used, with R(z) = max(0, z), so that the action of the convolutional layer 511 in the two-dimensional example is

$$\mathrm{x}^{(n)_b}\,[\mathrm{i,j}] = \mathrm{R}\left(\sum_a \left(K_{a,b} * x^{(n-1)a}\right)[i,j]\right) = \mathrm{R}\left(\sum_a \sum_{i'} \sum_{j'} K_{a,b}[i',j'] \cdot x^{(n-1)a}[i-i', j-j']\right)$$

It is also possible to use other activation functions, e.g., ELU (acronym for "Exponential Linear Unit"), LeakyReLU, Sigmoid, Tanh or Softmax.

In the displayed embodiment, the input layer 510 comprises 36 nodes 520, arranged as a two-dimensional 6x6 matrix. The first hidden node layer 512 comprises 72 nodes 522, arranged as two two-dimensional 6x6 matrices, each of the two matrices being the result of a convolution of the values of the input layer with a 3x3 kernel within the convolutional layer 511. Equivalently, the nodes 522 of the first hidden node layer 512 can be interpreted as arranged as a three-dimensional 2x6x6 matrix, wherein the first dimension correspond to the channel dimension.

The advantage of using convolutional layers 511 is that spatially local correlation of the input data can exploited by enforcing a local connectivity pattern between nodes of adjacent layers, in particular by each node being connected to only a small region of the nodes of the preceding layer.

A pooling layer 513 is a connection layer between an anterior node layer 512 (with node values x(n-1)) and a posterior node layer 514 (with node values x(n)). In particular, a pooling layer 513 can be characterized by the structure and the weights of the edges and the activation function forming a pooling operation based on a nonlinear pooling function f. For example, in the two-dimensional case the values x(n) of the nodes 524 of the posterior node layer 514 can be calculated based on the values x(n-1) of the nodes 522 of the anterior node layer 512 as

$$\mathrm{x}^{(n)_b}[\mathrm{i,j}] = \mathrm{f}(\mathrm{x}^{(n\text{-}1)}[\mathrm{id}_1, \mathrm{jd}_2], \,...,\, \mathrm{x}^{(n-1)b}[(i+1)\mathrm{d}_1\text{·}1, (j+1)\mathrm{d}_2\text{·}1])$$

In other words, by using a pooling layer 513 the number of nodes 522, 524 can be reduced, by re-placing a number d1·d2 of neighboring nodes 522 in the anterior node layer 512 with a single node 522 in the posterior node layer 514 being calculated as a function of the values of said number of neighboring nodes. In particular, the pooling function f can be the max-function, the average or the L2-Norm. In particular, for a pooling layer 513 the weights of the incoming edges are fixed and are not modified by training.

The advantage of using a pooling layer 513 is that the number of nodes 522, 524 and the number of parameters is reduced. This leads to the amount of computation in the network being reduced and to a control of overfitting.

In the displayed embodiment, the pooling layer 513 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 500 are fully connected layers 515. A fully connected layer 515 is a connection layer between an anterior node layer 514 and a posterior node layer 516. A fully connected layer 513 can be characterized by the fact that a majority, in particular, all edges between nodes 514 of the anterior node layer 514 and the nodes 516 of the posterior node layer are present, and wherein the weight of each of these edges can be adjusted individually.

In this embodiment, the nodes 524 of the anterior node layer 514 of the fully connected layer 515 are displayed both as two-dimensional matrices, and additionally as non-related nodes (indicated as a line of nodes, wherein the number of nodes was reduced for a better presentability). This operation is also denoted as "flattening". In this embodiment, the number of nodes 526 in the posterior node layer 516 of the fully connected layer 515 smaller than the number of nodes 524 in the anterior node layer 514. Alternatively, the number of nodes 526 can be equal or larger.

Furthermore, in this embodiment the Softmax activation function is used within the fully connected layer 515. By applying the Softmax function, the sum the values of all nodes 526 of the output layer 516 is 1, and all values of all nodes 526 of the output layer 516 are real numbers between 0 and 1. In particular, if using the convolutional neural network 500 for categorizing input data, the values of the output layer 516 can be interpreted as the probability of the input data falling into one of the different categories.

In particular, convolutional neural networks 500 can be trained based on the backpropagation algorithm. For preventing overfitting, methods of regularization can be used, e.g., dropout of nodes 520, ..., 524, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

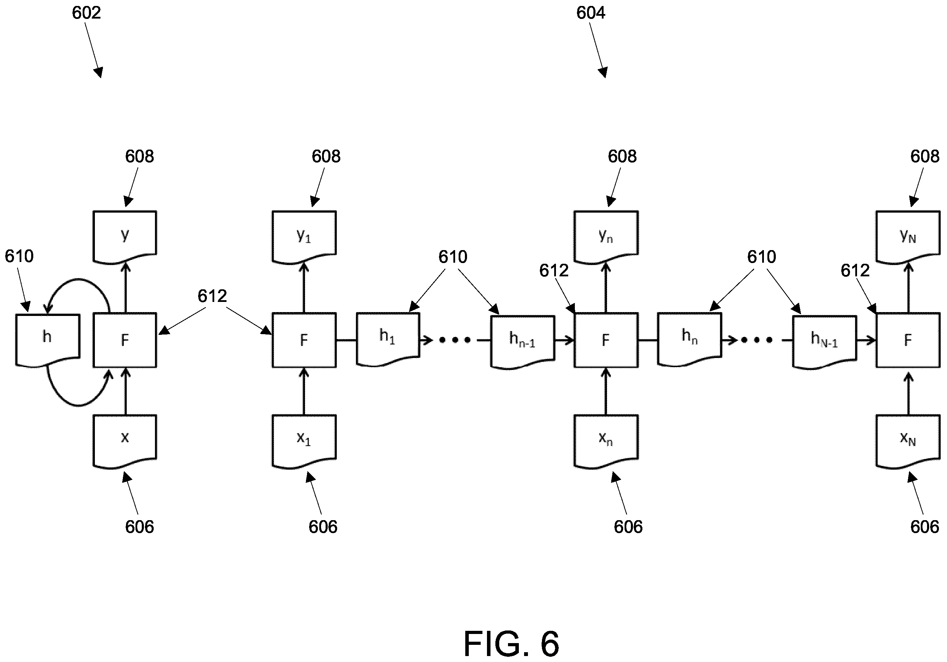

Figure 6 shows the schematic structure of a recurrent machine learning model F, both in a recurrent representation 602 and in an unfolded representation 604, that may be used to implement one or more machine learning models described herein. The recurrent machine learning model takes as input several input datasets $x, x_1, ..., x_N$ 606 and creates a corresponding set of output datasets $y, y_1, ..., y_N$ 608. Furthermore, the output depends on a so-called hidden vector $h, h_1, ..., h_N$ 610, which implicitly comprises information about input datasets previously used as input for the recurrent machine learning model F 612. By using these hidden vectors $h, h_1, ..., h_N$ 610, a sequentiality of the input datasets can be leveraged.

In a single step of the processing, the recurrent machine learning model F 612 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 612 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 612 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F^{(h)}(x_n, F^{(h)}(x_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 702 that may be used to implement systems, apparatuses, and methods described herein is depicted in Figure 7. Computer 702 includes a processor 704 operatively coupled to a data storage device 712 and a memory 710. Processor 704 controls the overall operation of computer 702 by executing computer program instructions that define such operations. The computer program instructions may be stored in data storage device 712, or other computer readable medium, and loaded into memory 710 when execution of the computer program instructions is desired. Thus, the method and workflow steps or functions of Figures 1 or 2 can be defined by the computer program instructions stored in memory 710 and/or data storage device 712 and controlled by processor 704 executing the computer program instructions. For example, the computer program instructions can be implemented as computer executable code programmed by one skilled in the art to perform the method and workflow steps or functions of Figures 1 or 2. Accordingly, by executing the computer program instructions, the processor 704 executes the method and workflow steps or functions of Figures 1 or 2. Computer 702 may also include one or more network interfaces 706 for communicating with other devices via a network. Computer 702 may also include one or more input/output devices 708 that enable user interaction with computer 702 (e.g., display, keyboard, mouse, speakers, buttons, etc.).

Processor 704 may include both general and special purpose microprocessors, and may be the sole processor or one of multiple processors of computer 702. Processor 704 may include one or more central processing units (CPUs), for example. Processor 704, data storage device 712, and/or memory 710 may include, be supplemented by, or incorporated in, one or more application-specific integrated circuits (ASICs) and/or one or more field programmable gate arrays (FPGAs).

Data storage device 712 and memory 710 each include a tangible non-transitory computer readable storage medium. Data storage device 712, and memory 710, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 708 may include peripherals, such as a printer, scanner, display screen, etc. For example, input/output devices 708 may include a display device such as a cathode ray tube (CRT) or liquid crystal display (LCD) monitor for displaying information to the user, a keyboard, and a pointing device such as a mouse or a trackball by which the user can provide input to computer 702.

An image acquisition device 714 can be connected to the computer 702 to input image data (e.g., medical images) to the computer 702. It is possible to implement the image acquisition device 714 and the computer 702 as one device. It is also possible that the image acquisition device 714 and the computer 702 communicate wirelessly through a network. In a possible embodiment, the computer 702 can be located remotely with respect to the image acquisition device 714.

Any or all of the systems, apparatuses, and methods discussed herein may be implemented using one or more computers such as computer 702.

One skilled in the art will recognize that an implementation of an actual computer or computer system may have other structures and may contain other components as well, and that Figure 7 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention as defined in the claims. Those skilled in the art could implement various other feature combinations without departing from the scope of the invention as defined in the claims.

The following is a list of non-limiting illustrative embodiments disclosed herein:

Illustrative embodiment 1. A computer-implemented method comprising: receiving one or more medical images of a patient; detecting one or more candidate nodules in the one or more medical images, each of the one or more candidate nodules associated with a nodule detection score; determining a malignancy score for each of the one or more candidate nodules; weighting the nodule detection score for each of the one or more candidate nodules based on its malignancy score; and outputting the weighted nodule detection scores.

Illustrative embodiment 2. The computer-implemented method of illustrative embodiment 1, wherein determining a malignancy score for each of the one or more candidate nodules comprises: for each respective candidate nodule of the one or more candidate nodules: determining a nodule type score for each of a plurality of nodule types for the respective candidate nodule; and determining the malignancy score for the respective candidate nodule based on the nodule type scores for the respective candidate nodule.

Illustrative embodiment 3. The computer-implemented method of illustrative embodiment 2, wherein: detecting one or more candidate nodules in the one or more medical images comprises determining a size of each of the one or more candidate nodules, and determining the malignancy score for the respective candidate nodule based on the nodule type scores for the respective candidate nodule comprises determining the malignancy score for the respective candidate nodule further based on the size of the respective candidate nodule.

Illustrative embodiment 4. The computer-implemented method of any of illustrative embodiments 2-3, wherein the plurality of nodule types comprises one or more of solid nodule, part-solid nodule, non-solid nodule, calcified nodule, perifissural nodule, or non-nodule.

Illustrative embodiment 5. The computer-implemented method of any of illustrative embodiments 1-4, further comprising: receiving user input defining visualization settings for presenting the one or more candidate nodules; filtering the one or more candidate nodules according to the visualization settings; and displaying the one or more filtered candidate nodules.

Illustrative embodiment 6. The computer-implemented method of illustrative embodiment 5, wherein the visualization settings comprise at least one of: one or more thresholds of the weighted nodule detection scores, one or more thresholds of a nodule size, a selection of one or more nodule types, or one or more thresholds of a malignancy scores.

Illustrative embodiment 7. The computer-implemented method of any of illustrative embodiments 1-6, wherein the one or more candidate nodules comprises one or more candidate pulmonary nodules.

Illustrative embodiment 8. An apparatus comprising: means for receiving one or more medical images of a patient; means for detecting one or more candidate nodules in the one or more medical images, each of the one or more candidate nodules associated with a nodule detection score; means for determining a malignancy score for each of the one or more candidate nodules; means for weighting the nodule detection score for each of the one or more candidate nodules based on its malignancy score; and means for outputting the weighted nodule detection scores.

Illustrative embodiment 9. The apparatus of illustrative embodiment 8, wherein the means for determining a malignancy score for each of the one or more candidate nodules comprises: for each respective candidate nodule of the one or more candidate nodules: means for determining a nodule type score for each of a plurality of nodule types for the respective candidate nodule; and means for determining the malignancy score for the respective candidate nodule based on the nodule type scores for the respective candidate nodule.

Illustrative embodiment 10. The apparatus of illustrative embodiment 9, wherein: the means for detecting one or more candidate nodules in the one or more medical images comprises means for determining a size of each of the one or more candidate nodules, and the means for determining the malignancy score for the respective candidate nodule based on the nodule type scores for the respective candidate nodule comprises means for determining the malignancy score for the respective candidate nodule further based on the size of the respective candidate nodule.

Illustrative embodiment 11. The apparatus of any of illustrative embodiments 9-10, wherein the plurality of nodule types comprises one or more of solid nodule, part-solid nodule, non-solid nodule, calcified nodule, perifissural nodule, or non-nodule.

Illustrative embodiment 12. The apparatus of any of illustrative embodiments 8-11, further comprising: means for receiving user input defining visualization settings for presenting the one or more candidate nodules; means for filtering the one or more candidate nodules according to the visualization settings; and means for displaying the one or more filtered candidate nodules.

Illustrative embodiment 13. The apparatus of illustrative embodiment 12, wherein the visualization settings comprise at least one of: one or more thresholds of the weighted nodule detection scores, one or more thresholds of a nodule size, a selection of one or more nodule types, or one or more thresholds of a malignancy scores.

Illustrative embodiment 14. The apparatus of any of illustrative embodiments 8-13, wherein the one or more candidate nodules comprises one or more candidate pulmonary nodules.

Illustrative embodiment 15. A non-transitory computer-readable storage medium comprising instructions which, when executed by a computer, cause the computer to carry out operations comprising: receiving one or more medical images of a patient; detecting one or more candidate nodules in the one or more medical images, each of the one or more candidate nodules associated with a nodule detection score; determining a malignancy score for each of the one or more candidate nodules; weighting the nodule detection score for each of the one or more candidate nodules based on its malignancy score; and outputting the weighted nodule detection scores.

Illustrative embodiment 16. The non-transitory computer-readable storage medium of illustrative embodiment 15, wherein determining a malignancy score for each of the one or more candidate nodules comprises: for each respective candidate nodule of the one or more candidate nodules: determining a nodule type score for each of a plurality of nodule types for the respective candidate nodule; and determining the malignancy score for the respective candidate nodule based on the nodule type scores for the respective candidate nodule.

Illustrative embodiment 17. The non-transitory computer-readable storage medium of illustrative embodiment 16, wherein: detecting one or more candidate nodules in the one or more medical images comprises determining a size of each of the one or more candidate nodules, and determining the malignancy score for the respective candidate nodule based on the nodule type scores for the respective candidate nodule comprises determining the malignancy score for the respective candidate nodule further based on the size of the respective candidate nodule.

Illustrative embodiment 18. The non-transitory computer-readable storage medium of any of illustrative embodiments 16-17, wherein the plurality of nodule types comprises one or more of solid nodule, part-solid nodule, non-solid nodule, calcified nodule, perifissural nodule, or non-nodule.

Illustrative embodiment 19. The non-transitory computer-readable storage medium of any of illustrative embodiments 15-18, further comprising: receiving user input defining visualization settings for presenting the one or more candidate nodules; filtering the one or more candidate nodules according to the visualization settings; and displaying the one or more filtered candidate nodules.

Illustrative embodiment 20. The non-transitory computer-readable storage medium of any of illustrative embodiments 15-19, wherein the visualization settings comprise at least one of: one or more thresholds of the weighted nodule detection scores, one or more thresholds of a nodule size, a selection of one or more nodule types, or one or more thresholds of a malignancy scores.

**Claims**

1. A computer-implemented method comprising:

   receiving (102) one or more medical images (202) of a patient;
   detecting (104, 204) one or more candidate nodules (206) in the one or more medical images, each of the one or more candidate nodules associated with a nodule detection score;
   determining (106, 214) a malignancy score (216) for each of the one or more candidate nodules;
   weighting (108, 218) the nodule detection score for each of the one or more candidate nodules based on its malignancy score; and
   outputting (110) the weighted nodule detection scores(220).

2. The computer-implemented method of claim 1, wherein determining a malignancy score for each of the one or more candidate nodules comprises:
   for each respective candidate nodule of the one or more candidate nodules:

   determining (210) a nodule type score (212) for each of a plurality of nodule types for the respective candidate nodule; and
   determining the malignancy score for the respective candidate nodule based on the nodule type scores for the respective candidate nodule.

3. The computer-implemented method of claim 2, wherein:

   detecting one or more candidate nodules in the one or more medical images comprises determining a size of each of the one or more candidate nodules, and
   determining the malignancy score for the respective candidate nodule based on the nodule type scores for the respective candidate nodule comprises determining the malignancy score for the respective candidate nodule further based on the size of the respective candidate nodule.

4. The computer-implemented method of claim 2 or 3, wherein the plurality of nodule types comprises one or more of solid nodule, part-solid nodule, non-solid nodule, calcified nodule, perifissural nodule, or non-nodule.

5. The computer-implemented method of any one of claims 1 - 4, further comprising:

   receiving user input defining visualization settings for presenting the one or more candidate nodules;
   filtering the one or more candidate nodules according to the visualization settings; and
   displaying the one or more filtered candidate nodules.

6. The computer-implemented method of claim 5, wherein the visualization settings comprise at least one of: one or more thresholds (302) of the weighted nodule detection scores, one or more thresholds (304) of a nodule size, a selection (306) of one or more nodule types, or one or more thresholds (308) of a malignancy scores.

7. The computer-implemented method of any one of claims 1 - 6, wherein the one or more candidate nodules comprises one or more candidate pulmonary nodules.

8. An apparatus comprising:

   means for receiving (102) one or more medical images (202) of a patient;
   means for detecting (104, 204) one or more candidate nodules (206) in the one or more medical images, each of the one or more candidate nodules associated with a nodule detection score;
   means for determining (106, 214) a malignancy score (216) for each of the one or more candidate nodules;
   means for weighting (108, 218) the nodule detection score for each of the one or more candidate nodules based on its malignancy score; and
means for outputting (110) the weighted nodule detection scores (220).

9. The apparatus of claim 8, wherein the means for determining a malignancy score for each of the one or more candidate nodules comprises:

for each respective candidate nodule of the one or more candidate nodules:

means for determining (210) a nodule type score (212) for each of a plurality of nodule types for the respective candidate nodule; and
means for determining the malignancy score for the respective candidate nodule based on the nodule type scores for the respective candidate nodule.

10. The apparatus of claim 9, wherein:

the means for detecting one or more candidate nodules in the one or more medical images comprises means for determining a size of each of the one or more candidate nodules, and
the means for determining the malignancy score for the respective candidate nodule based on the nodule type scores for the respective candidate nodule comprises means for determining the malignancy score for the respective candidate nodule further based on the size of the respective candidate nodule.

11. The apparatus of claim 9 or 10, wherein the plurality of nodule types comprises one or more of solid nodule, part-solid nodule, non-solid nodule, calcified nodule, perifissural nodule, or non-nodule.

12. The apparatus of any one of claims 8 - 11, further comprising:

means for receiving user input defining visualization settings for presenting the one or more candidate nodules;
means for filtering the one or more candidate nodules according to the visualization settings; and
means for displaying the one or more filtered candidate nodules.

13. The apparatus of claim 12, wherein the visualization settings comprise at least one of: one or more thresholds (302) of the weighted nodule detection scores, one or more thresholds (304) of a nodule size, a selection (306) of one or more nodule types, or one or more thresholds (308) of a malignancy scores.

14. The apparatus of any one of claims 8 - 13, wherein the one or more candidate nodules comprises one or more candidate pulmonary nodules.

15. A computer-program product comprising instructions which, when executed by a computer, cause the computer to carry out the method according to any one of claims 1 - 7.

# FIG. 1

100

```
┌─────────────────────────────────────────────────────────┐
│         Receive one or more medical images of a patient  │
│                           102                            │
└─────────────────────────────────────────────────────────┘
                            │
                            ▼
┌─────────────────────────────────────────────────────────┐
│   Detect one or more candidate nodules in the one or     │
│   more medical images, each of the one or more candidate │
│   nodules associated with a nodule detection score       │
│                           104                            │
└─────────────────────────────────────────────────────────┘
                            │
                            ▼
┌─────────────────────────────────────────────────────────┐
│   Determine a malignancy score for each of the one or    │
│   more candidate nodules                                 │
│                           106                            │
└─────────────────────────────────────────────────────────┘
                            │
                            ▼
┌─────────────────────────────────────────────────────────┐
│   Weight the nodule detection score for each of the one  │
│   or more candidate nodules based on its malignancy score│
│                           108                            │
└─────────────────────────────────────────────────────────┘
                            │
                            ▼
┌─────────────────────────────────────────────────────────┐
│         Output the weighted nodule detection scores      │
│                           110                            │
└─────────────────────────────────────────────────────────┘
```

200

**202** 3D CT scan

**204** Detection Model

**206** Candidate Nodules

The k-th candidate:
*location*
*size*
*probability*

**208** Extracted 3D nodule patch

**210** Type Classification

**212** Probability of each nodule type
solid nodule
part-solid nodule
non-solid nodule
calcified nodule
perifissural nodule
non-nodule

size

**214** Malignancy Classification

**216** Probability of malignancy
malignant
benign

**218** Malignancy-weighting Module

**220** Updated Nodules

The k-th candidate:
*location*
*size*
*updated probability*

**222** Visualization Module

**224**

FIG. 2

# FIG. 3

300

**Visualization setting**

**Detected nodule** — 302

   ◯ High-confident

   ◉ High & Median-confident (default)

   ◯ High & Median & Low-confident

**Nodule size** — 304

   ≥ 4mm

   Small                Large

**Nodule types** — 306

   ☑ Solid nodules

   ☑ Part-solid nodules

   ☑ Non-solid nodules

   ☐ Calcified nodules

   ☐ Perifissural nodules

**Malignant nodule** — 308

   ◯ High-confident

   ◉ High & Median-confident (default)

   ◯ High & Median & Low-confident

**Nodule display order** — 310

   ◯ Detection probability priority

   ◉ Malignancy probability priority

FIG. 4

EP 4 715 838 A1

FIG. 5

FIG. 6

# FIG. 7

702

Network interface 706

I/O 708

Processor 704

Storage 712

Memory 710

Image Acquisition Device 714

Europäisches
Patentamt

European
Patent Office

Office européen
des brevets

# EUROPEAN SEARCH REPORT

**Application Number**

EP 25 20 2919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 114 300 123 A (SSH VI XINJIANG MEDICAL TECH SHANGHAI CO LTD) 8 April 2022 (2022-04-08) | 1,5-8, 12-15 | INV. G16H30/40 |
| Y | * paragraphs [0007] - [0010], [0013], [0021], [0015]; claim 7 * | 2-4,9-11 | |
| Y | AU 2021 105 838 A4 (AFFILIATED ZHONGSHAN HOSPITAL DALIAN UNIV [CN]) 21 October 2021 (2021-10-21) * page 7, paragraph 1 * * page 1, paragraph 1 * | 2-4,9-11 | |
| A | CN 115 497 642 A (TIANJIN CANCER HOSPITAL TIANJIN MEDICAL UNIV CANCER INSTITUTE & HOSPIT) 20 December 2022 (2022-12-20) * the whole document * | 1-15 | |
| A | CN 117 476 212 A (SUN YAT SEN UNIV CANCER CENTER SUN YAT SEN UNIV AFFILIATED CANCER HOSP) 30 January 2024 (2024-01-30) * the whole document * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 23 January 2026 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 2919

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2026

| Patent document cited in search report | | Publication date | Patent family member(s) | Publication date |
|---|---|---|---|---|
| CN 114300123 | A | 08-04-2022 | NONE | |
| AU 2021105838 | A4 | 21-10-2021 | NONE | |
| CN 115497642 | A | 20-12-2022 | NONE | |
| CN 117476212 | A | 30-01-2024 | NONE | |

EPO FORM P0459

For more details about this annex : see Official Journal of the European Patent Office, No. 12/82

**EP 4 715 838 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 629023
- US 20230351601
- US 20240046466